# EUROPÄISCHE PATENTANMELDUNG

(11) **EP 2 253 373 A2**
(43) Veröffentlichungstag der Anmeldung: **24.11.2010**
(21) Anmeldenummer: 10162685.1
(22) Anmeldetag: 12.05.2010
(51) Int. Cl.: B01F 3/18, B01F 7/22, B01F 13/10, B01F 15/02, C12M 1/107

(54) **Anlage zur Beschickung eines Fermenters**

(30) Priorität: 19.05.2009 DE 202009004830 U
(71) Anmelder: Eder GmbH, 83104 Tuntenhausen (DE)
(72) Erfinder: Eckart, Gerhard, 84036, Landshut (DE)
(74) Vertreter: Gustorf, Gerhard

(57) **Zusammenfassung**

Die Anlage dient zur Beschickung eines Fermenters mit Biomasse, der mit einer Vorrichtung zum Mischen und Zerkleinern von pflanzlichen Abfällen verbunden ist. Gemäß der Erfindung ist vorgesehen, dass zwischen die Vorrichtung (10) und den Fermenter (12) ein Vorratsbehälter (14) geschaltet ist, der ein Rührwerk (30) aufweist und über eine kurze Beschickungsleitung (24) mit der Vorrichtung (10) zum Mischen und Zerkleinern und über eine Druck- und Saugleitung (28) mit dem Fermenter (12) verbunden ist.

## Beschreibung

Die Erfindung betrifft eine Anlage zur Beschickung eines Fermenters mit Biomasse, der mit einer Vorrichtung zum Mischen und Zerkleinern von pflanzlichen Abfällen verbunden ist.

Vorrichtungen zum Mischen und Zerkleinern von Schüttgut, insbesondere pflanzlichen Abfällen sind Gegenstand der Gebrauchsmuster 20 2004 002 546 und 20 2009 004 733 des Anmelders. Sie finden vorwiegend in der Landwirtschaft Anwendung, um beispielsweise Futter zu mischen und zu zerkleinern, etwa grob- oder langfasriges Silagegut, Heu, Stroh, Mineralfutter oder dergleichen, das dann über eine Austragöffnung in einem Stall dosiert verteilt werden kann.

Daneben finden diese Vorrichtungen auch Anwendung zur Beschickung eines Fermenters mit zerkleinerten pflanzlichen Abfällen in einer Biogasanlage. Hierbei war es bisher üblich, das zerkleinerte, pflanzliche Feststoffgemisch über eine schräg nach oben verlaufende Leitung mit Förderschnecke in den Fermenter zu transportieren, dessen Einfüllöffnung oberhalb der Vorrichtung zum Mischen und Zerkleinern liegt. Dabei hat es sich als Nachteil herausgestellt, dass die verhältnismäßig lange Förderschnecke aufgrund des grossen Durchsatzes von Feststoffgemisch in der Größenordnung von 100 m³/Tag eine hohe Belastung und damit einen großen Verschleiß der Förderschnecke verursacht, so dass diese regelmäßig nach etwa zwei Jahren Einsatzzeit ausgetauscht werden muss. Die erforderlichen Kosten für die Reparatur schlagen neben dem hohen Anschaffungspreis erheblich zu Buche.

Der Erfindung liegt die Aufgabe zugrunde, hier Abhilfe zu schaffen und eine Anlage der eingangs umrissenen Bauweise zur Verfügung zu stellen, bei der die Förderverbindung zwischen der Misch- und Zerkleinerungsvorrichtung und dem Fermenter erheblich vereinfacht und keinem nennenswerten Verschleiß mehr unterworfen ist.

Zur Lösung dieser Aufgabe ist vorgesehen, dass zwischen die Vorrichtung und den Fermenter ein Vorratsbehälter geschaltet ist, der ein Rührwerk aufweist und der über eine nur kurze Beschickungsleitung mit der Misch- und Zerkleinerungsvorrichtung verbunden ist, während er über eine Druck- und Saugleitung mit dem Fermenter verbunden ist. In Weiterbildung der Erfindung ist an den geschlossenen Vorratsbehälter ein Kompressor angeschlossen, der von Druckbetrieb auf Saugbetrieb und umgekehrt umschaltbar ist.

Mit der Erfindung wird der wesentliche Vorteil erzielt, dass zur Verbindung des Vorratsbehälters mit dem Fermenter keine mechanisch bewegten Teile wie beispielsweise eine Förderschnecke erforderlich sind, so dass der bisher hohe Verschleiß nahezu ausgeschlossen ist. Zur Förderung von Biomasse aus dem Vorratsbehälter in den Fermenter wird in dem geschlossenen Vorratsbehälter durch den Kompressor ein Druck erzeugt, der ausreicht, um die Biomasse über die Leitung in den Fermenter zu drücken. Wenn umgekehrt der Kompressor auf Saugbetrieb umschaltet, wird aus dem Fermenter größtenteils flüssige Biomasse abgesaugt und in den Vorratsbehälter zurücktransportiert, so dass das darin befindliche Feststoffgemisch mit Flüssigkeit, beispielsweise warmer Gülle mit einer Temperatur zwischen etwa 37 und 50° C angereichert wird. Dies erleichtert ein Vermischen innerhalb des Vorratsbehälters mit Hilfe des Rührwerks.

Das Rührwerk besteht nach einem Merkmal der Erfindung aus wenigstens einem Stabmischer, der von einem Elektromotor angetrieben wird.

In besonders vorteilhafter Weiterbildung der Erfindung ist der Vorratsbehälter auf Wiegeelementen abgestützt, denen elektronische Sensoren zugeordnet sind, die über eine Steuerleitung mit dem Kompressor und dem Dreiwegeventil verbunden sind. Auf diese Weise lässt sich das Ein-, Aus- und Umschalten des Kompressors in Abhängigkeit von der Befüllung des Vorratsbehälters automatisieren.

Die Erfindung ist nachstehend an zwei Ausführungsbeispielen erläutert, die in der Zeichnung dargestellt sind. Es zeigen:

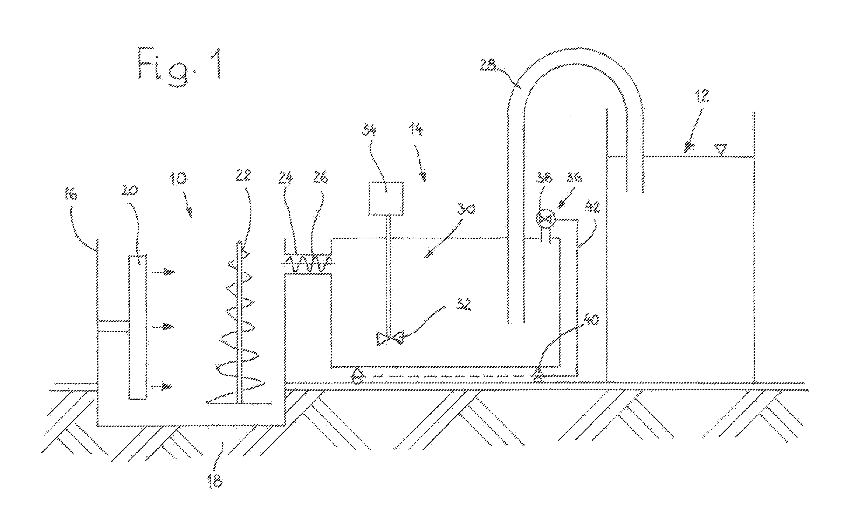

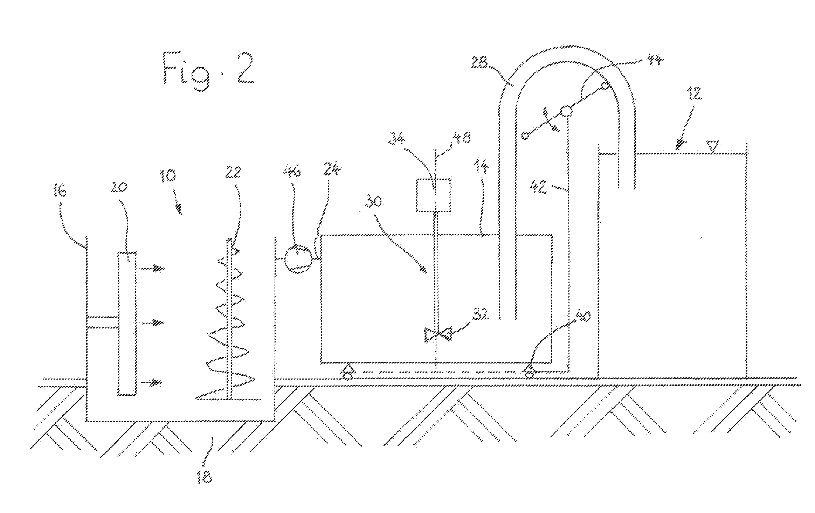

Figur 1 eine schematische Ansicht einer gemäß der Erfindung ausgebildeten Anlage und Figur 2 eine Variante der Figur 1.

Die in den Figuren schematisch dargestellte Anlage gemäß der Erfindung besteht aus einer Vorrichtung 10 zum Mischen und Zerkleinern, wie sie beispielsweise aus den eingangs genannten Gebrauchsmustern des Anmelders bekannt ist, aus einem Fermenter 12 und einem zwischen beiden angeordneten Vorratsbehälter 14, der die Form eines geschlossenen Kastens oder einer Kugel haben kann.

Die Vorrichtung 10 zum Mischen und Zerkleinern hat im dargestellten Ausführungsbeispiel einen oben offenen Aufnahmebehälter 16 für das zu mischende, pflanzliche Schüttgut, wobei dieser teilweise im Boden 18 eines Verarbeitungsbetriebs versenkt sein kann. In dem Aufnahmebehälter 16 ist ein Förderorgan 20 in Form eines Schiebers untergebracht, der das Gut in Richtung auf eine Mischschnecke 22 schiebt, die gleichzeitig eine Zerkleinerung des Gutes vornimmt und dieses dann über eine kurze Beschickungsleitung 24 in den Vorratsbehälter 14 transportiert. Im Beispiel der Figur 1 ist in der Beschickungsleitung 24 eine Schnecke 26 angeordnet.

Der geschlossene Vorratsbehälter 14 ist über eine Druck- und Saugleitung 28 mit dem Fermenter 12 verbunden, der über eine nicht weiter dargestellte Leitung auch mit Gülle versorgt werden kann. Der Vorratsbehälter 14 ist mit einem Rührwerk 30 ausgestattet, das aus wenigstens einem Stabmischer 32 besteht, der von einem Elektromotor 34 angetrieben wird. Um in dem Vorratsbehälter 14 den gewünschten Über- bzw. Unterdruck erzeugen zu können, ist im Beispiel der Figur 1 ein Kompressor 36 mit einem umschaltbaren Dreiwegeventil 38 vorgesehen.

Der Funktionsablauf dieser Anlage ist wie folgt:
Zunächst wird der Vorratsbehälter 14 über die Beschickungsleitung 24 mit Flüssigkeit und dann mit dem zerkleinerten Gut bis zu einer gewünschten Füllhöhe befüllt, beispielsweise bis zur Hälfte. Anschließend wird der Kompressor 36 über das Dreiwegeventil 38 auf Saugbetrieb umgestellt, so dass über die Leitung 28 warme Gülle aus dem Fermenter 12 oder einem anderen Güllebehälter angesaugt und in den Vorratsbehälter 14 überführt wird. Nach Erreichen der maximalen Füllhöhe wird der Kompressor 36 wieder abgeschaltet, worauf das Rührwerk 30 eingeschaltet wird und für eine intensive Mischung des Inhalts im Vorratsbehälter 14 sorgt. Sodann wird das Dreiwegeventil 38 auf Druckbetrieb umgestellt, so dass der Kompressor 36 die durchmischte Biomasse über die Leitung 28 in den Fermenter 12 drückt.

In der Zeichnung ist angedeutet, dass der Vorratsbehälter 14 auf Wiegeelementen 40 abgestützt ist, denen nicht weiter dargestellte elektrische Sensoren zugeordnet sind, die über eine Steuerleitung 42 mit dem Kompressor 36 und dem Dreiwegeventil 38 verbunden sind. Wenn der Vorratsbehälter 14 ein vorher festgelegtes Füllgewicht erreicht hat, betätigt er die Sensoren der Wiegeelemente 40, die dann ihrerseits einen Steuerbefehl an den Kompressor 36 und das Dreiwegeventil 38 geben, um den Druckbetrieb zum Überleiten der Biomasse in den Fermenter 12 einzuleiten.

Wenn in dem Vorratsbehälter 14 ein vorher festgelegtes Mindestgewicht erreicht ist, wird über die Sensoren die Druckförderung durch den Kompressor 36 abgeschaltet.

In Weiterbildung der Erfindung können die Sensoren der Wiegeelemente 40 auch mit dem Elektromotor 34 des Rührwerks 30 sowie mit den Arbeitselementen (Förderorgan 20 und Mischschnecke 22) innerhalb der Vorrichtung 10 zum Mischen und Zerkleinern verbunden sein, um auch deren Betrieb steuern zu können.

Im Ausführungsbeispiel der Figur 2 ist anstelle des Kompressors 36 eine Schlauchpumpe 44 vorgesehen, die der flexiblen Saugleitung 28 zugeordnet ist und in beiden Drehrichtungen angetrieben werden kann, um von Saugbetrieb auf Druckbetrieb umzustellen. Diese Umstellung erfolgt ebenfalls über die Steuerleitung 42. Der Vorteil der Schlauchpumpe 44 besteht darin, dass diese sehr robust ist; bei einem Verschleiß kann der flexible Schlauch der Saugleitung 28 rasch und einfach ausgetauscht werden.

In Abänderung der Figur 1 ist bei dieser Variante in die Beschickungsleitung 24 ein Fördergebläse 46 eingesetzt.

Ferner ist angedeutet, dass der Vorratsbehälter 14 aus einem stehenden Kreiszylinder besteht, in dessen Mittelachse 48 das Rührwerk 30 positioniert ist.

## Patentansprüche

1. Anlage zur Beschickung eines Fermenters mit Biomasse, der mit einer Vorrichtung zum Mischen und Zerkleinern von pflanzlichen Abfällen verbunden ist, **dadurch gekennzeichnet, dass** zwischen die Vorrichtung (10) und den Fermenter (12) ein Vorratsbehälter (14) geschaltet ist, der ein Rührwerk (30) aufweist und über eine kurze Beschickungsleitung (24) mit der Vorrichtung (10) zum Mischen und Zerkleinern und über eine Druck- und Saugleitung (28) mit dem Fermenter (12) verbunden ist.

2. Anlage nach Anspruch 1, dass an den geschlossenen Vorratsbehälter (14) ein Kompressor (36) angeschlossen ist, der von Druckbetrieb auf Saugbetrieb umschaltbar ist.

3. Anlage nach Anspruch 2, **dadurch gekennzeichnet, dass** der Kompressor (36) über ein Dreiwegeventil (38) von Druck- auf Saugbetrieb und umgekehrt umschaltbar ist.

4. Anlage nach Anspruch 1, **dadurch gekennzeichnet, dass** der flexiblen Saugleitung (28) eine Schlauchpumpe (44) zugeordnet ist.

5. Anlage nach Anspruch 3, **dadurch gekennzeichnet, dass** der Vorratsbehälter (14) auf Wiegeelementen (40) abgestützt ist, denen elektronische Sensoren zugeordnet sind, die über eine Steuerleitung (42) mit dem Kompressor (36) und dem Dreiwegeventil (38) verbunden sind.

6. Anlage nach Anspruch 4, **dadurch gekennzeichnet, dass** der Vorratsbehälter (14) auf Wiegeelementen (40) abgestützt ist, denen elektronische Sensoren zugeordnet sind, die über eine Steuerleitung (42) mit der Schlauchpumpe (44) verbunden sind.

7. Anlage nach Anspruch 5 oder 6, **dadurch gekennzeichnet, dass** die Steuerleitung (42) auch mit dem Rührwerk (30) verbunden ist.

8. Anlage nach einem der vorhergehenden Ansprüche, **dadurch gekennzeichnet, dass** das Rührwerk (30) aus wenigstens einem von einem Elektromotor (34) angetriebenen Stabmischer (32) besteht.

9. Anlage nach einem der vorhergehenden Ansprüche, **dadurch gekennzeichnet, dass** die Beschickungsleitung (24) eine kurze Förderschnecke (22) aufweist.

10. Anlage nach einem der Ansprüche 1 bis 8, **dadurch gekennzeichnet, dass** die Beschickungsleitung (24) ein Fördergebläse (46) aufweist.
